# EUROPÄISCHE PATENTSCHRIFT

(11) **EP 1 659 780 B1**
(45) Veröffentlichungstag und Bekanntmachung des Hinweises auf die Patenterteilung: **30.07.2008**
(21) Anmeldenummer: 05025491.1
(22) Anmeldetag: 23.11.2005
(51) Int. Cl.: H04N 5/225, A61B 1/04, H01L 27/148, H01L 27/146

(54) **Bildaufnehmermodul sowie Verfahren zum Zusammenbauen eines Bildaufnehmermoduls**
Image pickup device and method of construction of said image pickup device
Dispositif de prises de vues et methode de construction dudit dispositif

(30) Priorität: 23.11.2004 DE 102004056946
(43) Veröffentlichungstag der Anmeldung: 24.05.2006
(73) Patentinhaber: Karl Storz GmbH & Co. KG, 78532 Tuttlingen (DE)
(72) Erfinder: Ayrenschmalz, Robert, 84100 Niederaichbach (DE)
(74) Vertreter: Heuckeroth, Volker

(56) Entgegenhaltungen:
- DE-A1- 3 736 688
- US-A- 4 745 470
- US-A- 5 754 313
- US-A- 5 857 963
- US-A- 6 142 930
- US-A1- 2002 142 510
- US-A1- 2004 167 378
- US-A1- 2004 263 680
- PATENT ABSTRACTS OF JAPAN Bd. 2000, Nr. 21, 3. August 2001 (2001-08-03) & JP 2001 104247 A (OLYMPUS OPTICAL CO LTD), 17. April 2001 (2001-04-17)

## Beschreibung

Die Erfindung betrifft ein Bildaufnehmermodul, insbesondere für ein Endoskop oder eine Miniaturkamera, mit einem elektronischen Bildsensor, der eine Mehrzahl von Kontaktfingern aufweist, die in zwei Reihen an gegenüberliegenden Seiten angeordnet sind und jeweils eine Länge aufweisen, und mit einer Platine, mit der die Kontaktfinger elektrisch kontaktiert sind, wobei die Platine zumindest drei Abschnitte aufweist, von denen sich ein erster und ein zweiter Abschnitt voneinander beabstandet im Wesentlichen quer zur Ebene des Bildsensors und ein dritter Abschnitt im Wesentlichen parallel zur Ebene des Bildsensors erstrecken, so dass zwischen dem ersten, zweiten und dritten Abschnitt ein Raum vorhanden ist, wobei der Bildsensor an dem dem dritten Abschnitt abgewandten Ende des ersten und zweiten Abschnitts angeordnet ist, und wobei zumindest eine Reihe der Kontaktfinger des Bildsensors entlang einer Außenseite des ersten oder zweiten Abschnitts verläuft.

Die Erfindung betrifft ferner ein Verfahren zum Zusammenbauen eines Bildaufnehmermoduls, wobei ein elektronischer Bildsensor und eine Platine bereitgestellt werden, wobei der Bildsensor eine Mehrzahl von Kontaktfingern aufweist, die in zwei Reihen an gegenüberliegenden Seiten angeordnet sind und eine Länge aufweisen, wobei die Platine zumindest drei Abschnitte aufweist, von denen sich ein erster und ein zweiter Abschnitt voneinander beabstandet im Wesentlichen in einer ersten Richtung und ein dritter Abschnitt in einer zweiten Richtung im Wesentlichen quer zur ersten Richtung erstrecken, so dass zwischen dem ersten, zweiten und dritten Abschnitt ein Raum vorhanden ist, wobei der Bildsensor an dem dem dritten Abschnitt abgewandten Ende des ersten und zweiten Abschnitts angebracht wird, die Kontaktfinger zumindest einer Reihe entlang einer Außenseite des ersten oder des zweiten Abschnitts angeordnet und mit der Platine elektrisch kontaktiert werden.

Ein derartiges Bildaufnehmermodul sowie ein derartiges Verfahren sind aus DE-A-199 24 189 bekannt.

Ein derartiges elektronisches Bildaufnehmermodul findet allgemein in der Videoaufnahmetechnik Anwendung. Neben der Verwendung in Videokameras werden solche elektronische Bildaufnehmermodule in möglichst miniaturisierter Bauform insbesondere auch in Endoskopen für technische oder medizinische Zwecke eingesetzt. Ein solches Endoskop bzw. Videoendoskop ist beispielsweise aus US 5,754,313 bekannt.

Ein Bildaufnehmermodul umfasst allgemein einen elektronischen Bildsensor bzw. Bildaufnehmer, der auf ihn einfallendes Licht in ein elektrisches Signal umwandelt. Allgemein sind solche elektronischen Bildsensoren in CCD- oder CMOS-Technologie ausgeführt.

So werden derzeit miniaturisierte Bildsensoren bevorzugt, die in TAB (tape automated bonding) -Technologie gefertigt sind. Derartige Bildsensoren weisen in zwei Reihen an gegenüberliegenden Seiten des Bildsensors angeordnete Kontaktfinger auf, die mit der Platine des Bildaufnehmermoduls kontaktiert werden.

Die Platine des Bildaufnehmermoduls dient nicht nur der Kontaktierung des Bildsensors, sondern, wie bei dem aus DE-A-199 24 189 bekannten Bildaufnehmermodul, auch zur Aufnahme elektronischer Bauelemente, wie Transistoren oder Kondensatoren, die Teile der Steuerelektronik des Bildsensors darstellen.

Bei dem eben genannten bekannten Bildaufnehmermodul ist die Platine aus einer entlang flexibler Verbindungsabschnitte faltbaren einteiligen Platte gebildet, die sich zu einem quaderförmigen, im Querschnitt im Wesentlichen U-förmigen Körper falten lässt. Der Platinenkörper weist im gefalteten Zustand zwei sich im Wesentlichen quer zum Bildsensor erstreckende und voneinander beabstandete Abschnitte und einen dritten Abschnitt auf, der im Wesentlichen parallel zum Bildsensor verläuft, wobei der Bildsensor an dem Ende des ersten und zweiten Abschnitts des Platinenkörpers angebracht ist, das von dem dritten Abschnitt beabstandet ist. Der dritte Abschnitt dieser Platine des bekannten Bildaufnehmermoduls dient der Durchführung eines von der Platine wegführenden elektrischen Kabels, und insbesondere der Zugentlastung dieses Kabels. Elektronische Bauelemente für die Steuerelektronik des Bildsensors sind dagegen auf einem vierten Abschnitt der Platine innenseitig angebracht, auf dessen Außenseite der Bildsensor angeordnet ist.

Der Einsatz solcher Bildaufnehmermodule in Endoskopen ist erst durch die Miniaturisierung der Bildsensoren und der Fortschritte in der Mikrotechnologie möglich geworden. In einem Endoskop ist das Bildaufnehmermodul üblicherweise in der distalen, d.h. dem Patienten zugewandten Spitze des Endoskopschafts angeordnet, wie dies beispielsweise in US 5,754,313 beschrieben ist. Das Bildaufnehmermodul ersetzt dabei die bei "klassischen" Endoskopen vorgesehene Bildübertragungsoptik, die aus einer Reihenanordnung von Linsen gebildet wird. Anstatt das distalseitig empfangene Bild mittels eines optisch abbildenden Linsensystems nach proximal, d.h. zum patientenfernen Ende zu übertragen, werden bei einem Bildaufnehmermodul die optischen Lichtsignale in elektrische Signale umgewandelt und über zumindest ein Kabel bzw. in der Regel ein Kabelsystem nach proximal zur Kamerakontrolleinheit übertragen.

Bei Endoskopen besteht stets die Forderung nach einem möglichst geringen Querschnitt der Außenkontur des Schafts. Dementsprechend müssen die verwendeten Bildaufnehmermodule, um in einem derartigen Schaft Platz zu finden, einen möglichst geringen Außenquerschnitt aufweisen. Beispielsweise beträgt der Schaftdurchmesser bei einem Videoendoskop für medizinische Zwecke nur wenige mm, auf jeden Fall weniger als 10 mm. Dies bedeutet, dass die Abmessungen des miniaturisierten Bildaufnehmermoduls so gering wie nur möglich ausgeführt sein sollten, d.h. möglichst kleiner als 6 mm. Eine kompakte Bauweise eines Bildaufnehmermoduls ist daher erwünscht.

Andererseits sollte das Bildaufnehmermodul in integrierter Bauweise eine Reihe von für die Funktion des Bildsensors erforderlichen Bauteilen (bspw. Verstärker) enthalten, so dass das Bildaufnehmermodul lediglich noch mit einem üblicherweise mehradrigen Kabel verbunden werden muss, das im Fall der Verwendung des Bildaufnehmermoduls in einem Endoskop in dessen distaler Spitze die Spannungsversorgung und Signalübertragung zwischen dem Bildaufnehmermodul und einer Steuerschaltung (Kamerakontrolleinheit) am proximalen Ende des Endoskops oder in einem externen Gerät herstellt.

Das aus US 5,754,313 bekannte Bildaufnehmermodul weist zwei separate Platinen, d.h. nicht eine einstückige einzelne Platine zur Kontaktierung des Bildsensors auf, wobei die beiden Platinen in üblicher Weise elektronische Miniaturbauteile aufnehmen und zur Kontaktierung des wegführenden Kabels bzw. Kabelsystems dienen. Die beiden Platinen verlaufen dabei parallel zueinander und etwa rechtwinklig zur Fläche des Bildsensors. Da die Signalelektronik auf den beiden Platinen nicht unabhängig funktionieren kann, muss eine elektrische Verbindung, beispielsweise in Form von Leitungen oder einer Verbindungsplatine, zusätzlich integriert werden, wodurch der Montageaufwand dieses bekannten Bildaufnehmermoduls erhöht ist. Der Raum zwischen den beiden separaten Platinen ist mit einem aushärtenden Kunststoff ausgefüllt. Das von der Platine weg führende Kabel ist dabei auf der Außenseite der beiden Platinen mit diesen kontaktiert, ebenso sind die Kontaktfinger des Bildsensors auf der Außenseite der beiden Platinen distalseitig der Kontaktierungsstellen des wegführenden Kabels mit den Platinen kontaktiert.

Auch dieses bekannte Bildaufnehmermodul weist somit eine Länge in Querrichtung zum Bildsensor auf, die die Seitenabmessungen des Bildsensors deutlich übersteigt.

Eine weitere Anforderung an ein derartiges Bildaufnehmermodul besteht darin, dass sich das Bildaufnehmermodul kostengünstig und mit geringem Zeitaufwand herstellen lassen sollte, insbesondere sollte der Zusammenbau der einzelnen Komponenten des Bildaufnehmermoduls trotz der Miniaturisierung und Kompaktheit des Bildaufnehmermoduls möglichst einfach durchzuführen sein.

In US 4,745,471 ist ein weiteres Bildaufnehmermodul beschrieben, bei dem der Bildsensor auf einer einstückigen, im Querschnitt U-förmigen Platine angeordnet ist. Der Bildsensor ist dabei in die U-förmige Nut der Platine eingebettet, wodurch zwar eine in Richtung quer zum Bildsensor sehr kurze Baulänge erreicht wird, jedoch müssen elektronische Bauelemente bei diesem bekannten Bildaufnehmermodul dann auf der Außenseite der Platine angebracht und kontaktiert werden, wodurch diese Bauteile wenig gegen äußere Einflüsse geschützt sind.

Aus dem Dokument US 2002/0142510 A1 ist ein Bildaufnehmermodul bekannt, bei dem der Bildsensor mit einem LSI-Chip elektrisch kontaktiert wird, d.h., dieses bekannte Bildaufnehmermodul weist keine Platine auf, mit der der Bildsensor kontaktiert ist. Auf der dem Bildsensor zugewandten Seite weist der LSI-Chip an seiner Oberfläche elektronische Bauelemente wie Transistoren auf. In einem Ausführungsbeispiel sind die Kontaktfinger des Bildsensors über zusätzliche Drähte mit der Unterseite des LSI-Chips kontaktiert. In diesem Fall sind der Bildsensor und der LSI-Chip Rücken an Rücken aneinander angeordnet.

Aus dem Dokument US 5,857,963 ist ein Bildaufnehmermodul bekannt, bei dem die einzelnen Kontaktfinger des Bildsensors, der in TAB-Ausführung ausgebildet ist, mit einer Platine kontaktiert sind, die die Form eines T aufweist. Diese Platine weist drei Abschnitte auf, von denen sich zwei quer zur Ebene des Bildsensors erstrecken, wobei ein dritter Abschnitt sich parallel zur Ebene des Bildsensors erstreckt und der Bildsensor an diesem dritten Abschnitt angeordnet ist, wobei die Kontaktfinger des Bildsensors unmittelbar mit dem dritten Abschnitt kontaktiert sind.

Ein Bildaufnehmermodul für ein Stereo-Videoendoskop ist in US 2004/0167378 A1 beschrieben, das entsprechend zwei Bildsensoren aufweist, die in TAB-Ausführung ausgebildet sind, wobei die flexible Platine in Richtung quer zur Ebene des Bildsensors lang baut und innenseitig elektronische Bauelemente trägt. In Richtung quer zur Ebene des Bildsensors ist die Platine länger als die Kontaktfinger des bzw. der Bildsensoren.

In JP 2001 104247 A ist allgemein ein Bildaufnehmermodul mit einem Bildsensor in TAB-Ausführung beschrieben.

In DE 37 36 688 A1 ist ein Bildaufnehmermodul beschrieben, dessen Bildsensor mit einer Platine kontaktiert ist, wobei Platinen in verschiedenen Ausgestaltungen offenbart werden. Die Platinen sind bei allen Ausführungsbeispielen in Form einzelner Platten ausgebildet, die sich quer zur Ebene des Bildsensors von diesem weg erstrecken. In manchen Ausführungsbeispielen sind zwei derartiger Platinen parallel zueinander angeordnet.

Dokument US 6,142,930 offenbart ein Bildaufnehmermodul, insbesondere für ein Endoskop oder eine Miniaturkamera, mit einem elektronischen Bildsensor, der eine Mehrzahl von Kontaktfingern aufweist, die in zwei Reihen an gegenüberliegenden Seiten angeordnet sind und jeweils eine Länge aufweisen, und mit einer Platine, mit der der die Kontaktfinger elektrisch kontaktiert sind, wobei die Platine zumindest drei miteinander verbundene Abschnitte aufweist, von denen sich ein erster und ein zweiter Abschnitt voneinander beabstandet im Wesentlichen quer zur Ebene des Bildsensors und ein dritter Abschnitt im Wesentlichen parallel zur Ebene des Bildsensors erstrecken, so dass zwischen dem ersten, zweiten und dritten Abschnitt ein Raum vorhanden ist, wobei der Bildsensor an dem dem dritten Abschnitt abgewandten Ende des ersten und zweiten Abschnitts angeordnet ist, und wobei zumindest eine Reihe der Kontaktfinger des Bildsensors entlang einer Außenseite des ersten oder zweiten Abschnitts verläuft wobei der erste Abschnitt der zweite Abschnitt und der dritte Abschnitt miteinander verbunden sind, und die Länge des ersten Abschnitts und/oder des zweiten Abschnitts kleiner als oder gleich der Länge zumindest eines Teils der Kontaktfinger ist, und ein Raum zwischen dem ersten, zweiten und dritten Abschnitt der Platine vorhanden ist in dem zumindest ein elektronisches Bauelement für die Steuerelektronik des Bildsensors vorhanden ist.

Dieses Modul bildet den nächsten Stand der Technik für die Erfindung.

Der Erfindung liegt die Aufgabe zugrunde, das Bildaufnehmermodul dahingehend weiterzubilden, dass es insgesamt kompakt baut, insbesondere in Richtung quer zum Bildsensor eine möglichst geringe Baulänge aufweist, und dass das Bildaufnehmermodul leicht herstellbar ist.

Der Erfindung liegt des Weiteren die Aufgabe zugrunde, ein Verfahren zum Zusammenbauen eines Bildaufnehmermoduls anzugeben, das einfach durchführbar ist.

Erfindungsgemäß wird diese Aufgabe dadurch gelöst, dass die Platine starr ist und der erste Abschnitt, der zweite Abschnitt und der dritte Abschnitt ungelenkig und einstückig miteinander verbunden sind, und dass der Raum zwischen dem ersten, zweiten und dritten Abschnitt der Platine in Form einer Nut oder Einkerbung ausgebildet ist.

Die Platine des erfindungsgemäßen Bildaufnehmermoduls ist somit durch einen einstückigen festen Körper gebildet, der eine Einkerbung oder eine Nut aufweist, indem zumindest ein elektronisches Bauelement, vorzugsweise mehrere elektronische Bauelemente, für die distale Steuerelektronik des Bildsensors angeordnet sind, beispielsweise ein Transistor (Verstärker) und ein Kondensator. Auf der "offenen" Seite der Platine bzw. des U-förmigen Querschnitts ist der Bildsensor angebracht, beispielsweise auf die freien Enden des ersten Abschnitts und des zweiten Abschnitts der Platine aufgeklebt. Auf diese Weise kann das Bildaufnehmermodul mit einer insgesamt quader- oder vorzugsweise würfelförmigen Außenkontur ausgebildet werden, deren Kantenlänge der Kantenlänge des Bildsensors in dessen Ebene entspricht. Durch die ungelenkige oder starre Verbindung der drei Abschnitte miteinander wird eine insgesamt sehr stabile Bauweise des Bildaufnehmermoduls geschaffen, wobei die starre Platine gleichzeitig als Gehäuse dient und damit Schutz für die darin befindlichen Komponenten bietet. Des Weiteren ist die Länge der Platine in Richtung quer zum Bildsensor so klein gewählt, dass sie nicht größer als die Länge der Kontaktfinger des Bildsensors ist, was den weiteren Vorteil hat, dass die Kontaktfinger, die entlang zumindest einer Außenseite des ersten oder zweiten Abschnitts verlaufen, sogar auf der Außen- bzw. Unterseite des dritten Abschnitts mit der Platine kontaktiert werden können, wie dies in einer bevorzugten Ausgestaltung vorgesehen ist.

In einer bevorzugten Ausgestaltung greifen die entlang dem ersten oder zweiten Abschnitt verlaufenden Kontaktfinger des Bildsensors zumindest teilweise auf eine Außenseite des dritten Abschnitts der Platine und sind an dieser mit der Platine kontaktiert.

Der Vorteil dieser Maßnahme besteht darin, dass in Verbindung mit der festen Ausgestaltung der Platine die um die Außenseite des dritten Abschnitts greifenden Kontaktfinger zum einen die Gesamtstabilität des Bildaufnehmermoduls weiter erhöhen, zum anderen aber auch die Befestigung des Bildaufnehmermoduls an der Platine sichern. Dabei kann es vorgesehen sein, dass nur einzelne der Gesamtzahl an Kontaktfingern die Außenseite des dritten Abschnitts der Platine umgreifen, während die übrigen Kontaktfinger an der Außenseite des ersten und/oder zweiten Abschnitts enden. Die Kontaktierung zumindest eines Teils der Kontaktfinger oder vorzugsweise aller Kontaktfinger des Bildsensors an der Außenseite des dritten Abschnitts, wie in einer weiteren bevorzugten Ausgestaltung vorgesehen ist, hat den Vorteil, dass die Querschnittsabmessung der Außenkontur des Bildaufnehmermoduls nicht durch seitliche Kontaktierungsstellen erhöht wird, sondern dass die Außenseiten des ersten und zweiten Abschnitts im Wesentlichen bündig mit den entsprechenden Seitenkanten des Bildsensors abschließen können.

In diesem Sinne ist es weiterhin bevorzugt, wenn auf der Außenseite des ersten und/oder zweiten Abschnitts Vertiefungen zur Aufnahme der Kontaktfinger ausgebildet sind.

Diese Maßnahme trägt weiterhin dazu bei, dass die Querschnittsabmessungen des Bildaufnehmermoduls bei gleichzeitig maximal nutzbarer Querschnittsabmessung der Platine, die der Querschnittsabmessung des Bildsensors entspricht, die Kontaktfinger nicht zu einer weiteren Querschnittsvergrößerung führen, da diese in den Vertiefungen auf den Außenseiten des ersten bzw. zweiten Abschnitts eingebettet sind.

In einer weiteren bevorzugten Ausgestaltung sind in dem Raum zumindest zwei elektronische Bauelemente in Richtung von dem dritten Abschnitt zu dem Bildsensor übereinanderliegend angeordnet.

Bei dieser Maßnahme wird der Raum, d.h. die oben erwähnte Nut bzw. Einkerbung der Platine, bei gleichzeitig kompakter Bauweise des Moduls optimal für die Aufnahme von elektronischen Bauelementen genutzt. Die elektronischen Bauelemente werden dabei ausgehend von dem dritten Abschnitt, auf dem beispielsweise ein Transistor befestigt ist, in Stapelanordnung aufeinander angeordnet und aneinander befestigt. Somit wird der Innenraum der Platine dreidimensional genutzt, was eine Verringerung der Abmessungen der Außenkontur der Platine ermöglicht.

In einer weiteren bevorzugten Ausgestaltung ist das zumindest eine elektronische Bauelement mit zumindest einem Leiterdraht an einer Stelle des Raums, die von dem ersten, zweiten und dritten Abschnitt beabstandet ist, kontaktiert.

Bei dieser Maßnahme wird beispielsweise, wenn es sich bei dem elektronischen Bauelement um einen Transistor handelt, der üblicherweise drei Kontaktfinger aufweist, jeder einzelne Kontaktfinger mittels eines Leiterdrahts mit der Platine verbunden. Das Anbringen des Leiterdrahts an dem jeweiligen Kontaktfinger an einer Stelle des Raums bzw. der Nut oder der Einkerbung, die von dem ersten, zweiten und dritten Abschnitt beabstandet ist, hat den Vorteil, dass der Raum zwischen den drei Abschnitten, wie zuvor erwähnt, optimal in seiner dreidimensionalen Erstreckung genutzt wird, was dazu beiträgt, dass die drei Abschnitte der Platine selbst hinsichtlich ihrer zweidimensionalen Abmessungen klein gehalten sein können.

Dabei ist es weiterhin bevorzugt, wenn der zumindest eine Leiterdraht ausgehend von dem zumindest einen elektronischen Bauelement zumindest teilweise durch eine sich durch den ersten oder zweiten Abschnitt erstreckende Bohrung geführt ist, die an der Außenseite des dritten Abschnitts mündet.

Hierbei ist von Vorteil, dass auch die erforderliche Kontaktierung des zumindest einen elektronischen Bauelements, das im Innenraum der Platine angeordnet ist, auf der Außenseite der Platine, insbesondere auf der Außenseite des dritten Abschnitts, erfolgt, was die Kontaktierung, beispielsweise mittels einer Lötverbindung, besonders einfach macht, weil die Außenseite des dritten Abschnitts leicht zugänglich ist. Die Durchführung des Leiterdrahts durch eine Bohrung durch den ersten, zweiten oder dritten Abschnitt hat den Vorteil, dass die Durchkontaktierung auf die Außenseite des dritten Abschnitts durch das Material der Platine erfolgt und somit keinen zusätzlichen Platz benötigt. Auch unter Isolationsgesichtspunkten ist diese Maßnahme vorteilhaft, insbesondere, wenn die Platine insgesamt aus einem isolierenden Material, beispielsweise einem harten Kunststoff, gefertigt ist, da dann mehrere solcher Bohrungen vorgesehen sein können, die voneinander beabstandet sind, so dass jeder Leiterdraht durch eine eigene Bohrung zur Außenseite des dritten Abschnitts von den anderen Leiterdrähten isoliert geführt werden kann. Die Bohrungen können zumindest teilweise metallisiert sein, so dass die Leiterdrähte nicht vollständig durch die Bohrungen geführt werden müssen, sondern die elektrische Leitung über die Metallisierung der Bohrungen zur Außenseite des dritten Abschnitts erfolgt.

In einer weiteren bevorzugten Ausgestaltung ist der verbleibende freie Teil des Raums zwischen dem ersten, zweiten und dritten Abschnitt und dem zumindest einen elektronischen Bauelement mit einer isolierenden aushärtbaren Füllmasse ausgefüllt.

Die aushärtende Füllmasse hat zum einen den vorteil, dass sie nach dem Aushärten die gesamte Anordnung aus Bildsensor, Platine und dem zumindest einen elektronischen Bauelement weiter stabilisiert bzw. verstärkt, andererseits dient sie auch vorteilhafterweise der Isolation der Leiterdrähte, die zur Kontaktierung des Bauelements bzw. der Bauelemente vorhanden sind.

In einer weiteren bevorzugten Ausgestaltung ist die Platine aus einem quaderförmigen, insbesondere etwa würfelförmigen Vollmaterial gefertigt, und der Raum zwischen dem ersten, zweiten und dritten Abschnitt ist durch eine Materialausnehmung aus dem Vollmaterial gebildet.

Diese Maßnahme hat zum einen den Vorteil, dass sich die Platine durch die Materialausnehmung in einem besonders einfach durchführbaren Vorgang leicht fertigen lässt, zum anderen weist die fertige Platine auch die gewünschte hohe Stabilität auf, da sie aus einem Vollmaterial als Ausgangsmaterial gebildet ist.

In einer weiteren bevorzugten Ausgestaltung weist der Raum zwischen dem ersten, zweiten und dritten Abschnitt im Querschnitt etwa die Form eines T auf, wobei sich der breitere Abschnitt des Raums an dem dem Bildsensor zugewandten Ende des ersten und zweiten Abschnitts befindet.

Durch die T-förmige Ausgestaltung des Raums zwischen den drei Abschnitten entsteht eine Stufe auf der Innenseite des ersten und zweiten Abschnitts, was die Durchführung von Leiterdrähten durch das Material des ersten und zweiten Abschnitts zur Kontaktierung von Bauelementen, die sich in dem Raum zwischen den drei Abschnitten befinden, erleichtert. Des weiteren wird auch die Gefahr vermieden, dass die Leiterdrähte unerwünscht Kontakt zu dem Bildsensor erhalten, da dieser von der Stufe in dem ersten und zweiten Abschnitt beabstandet ist, während die Leiterdrähte auf Höhe der Stufe positioniert sind.

In einer weiteren bevorzugten Ausgestaltung ist der Bildsensor unmittelbar an den Enden des ersten und zweiten Abschnitts der Platine befestigt.

Diese Maßnahme führt nicht zu einer verringerten Baulänge des Bildaufnehmermoduls, sondern trägt auf vorteilhafte Weise zur Stabilisierung des gesamten Bildaufnehmeimoduls bei und vereinfacht die mechanische verbindung des Bildsensors mit der Platine.

Wie bereits zuvor erwähnt, ist es weiter bevorzugt, wenn sich durch das Material zumindest eines von dem ersten und dem zweiten Abschnitt der Platine, von der Außenseite des dritten Abschnitts ausgehend, eine Mehrzahl an Bohrungen oder Leitern erstrecken, die an einer Innenseite des dritten Abschnitts, des ersten und/oder zweiten Abschnitts münden bzw. enden.

Die Bohrungen oder Leitern, die sich durch das Material der drei Abschnitte erstrecken, haben den Vorteil einer leichten Durchkontaktierung vom Innenraum der Platine zur Außenseite des dritten Abschnitts. Diese Ausgestaltung ist insbesondere mit der zuvor erwähnten T-förmigen Ausgestaltung des Raums zwischen den drei Abschnitten vorteilhaft, wobei die Bohrungen im ersten und/oder zweiten Abschnitt dann vorteilhafterweise in der durch die T-Struktur des Raums gebildeten Stufe im ersten bzw. zweiten Abschnitt münden.

Im Sinne einer miniaturisierten Ausgestaltung des erfindungsgemäßen Bildaufnehmermoduls, insbesondere wenn es für die Verwendung in einem Endoskop vorgesehen ist, ist es vorgesehen, dass das Bildaufnehmermodul etwa würfelförmig ausgebildet ist und Seitenabmessungen von etwa 2 mm x 2 mm oder kleiner aufweist.

In einer weiteren bevorzugten Ausgestaltung ist die Platine mit einem mehradrigen Kabel verbunden, wobei das Kabel an der Außenseite des dritten Abschnitts mit der Platine kontaktiert ist.

Hierbei ist von Vorteil, dass die Kontaktierung des von der Platine weg führenden Kabels mit der Platine besonders einfach bewerkstelligbar ist, da die Außenseite des dritten Abschnitts für den Kontaktierungsvorgang besonders gut zugänglich ist. Dabei ist es weiterhin bevorzugt, wenn das Kabel lösbar mit der Platine verbunden ist.

Die lösbare Verbindung des Kabels mit der Platine hat den Vorteil, dass das Bildaufnehmermodul zu wartungszwecken leicht vom Kabel getrennt und beispielsweise damit auch leicht aus einem Endoskop ausgebaut werden kann. Die lösbare Verbindung kann beispielsweise dadurch realisiert werden, dass das Kabel mit einer weiteren Platine kontaktiert wird, die komplementär zu der Außenseite des dritten Abschnitts der Platine ist. Das Kabel wird dann mit dieser Zusatzplatine fest verbunden, und die Zusatzplatine wird dann mit der Außenseite des dritten Abschnitts durch bloße Berührung kontaktiert und beispielsweise über einen leitfähigen Kleber oder über eine leicht lösbare Lötung verbunden.

In einer weiteren bevorzugten Ausgestaltung des Bildaufnehmermoduls ist eine Reihe der Kontaktfinger des Bildsensors abgelängt und auf einer Außenseite des ersten oder zweiten Abschnitts mit der Platine kontaktiert.

Hierbei ist von Vorteil, dass auf der Außenseite des dritten Abschnitts der Platine mehr Fläche zur Kontaktierung des wegführenden Kabelsystems zur Verfügung steht.

In einer weiteren bevorzugten Ausgestaltung verlaufen der erste und/oder der zweite Abschnitt zumindest außenseitig zum dritten Abschnitt hin zur Mittelachse des dritten Abschnitts schräg.

Aufgrund dieser Maßnahme verjüngt sich das Bildaufnehmermodul außenseitig vom Bildsensor aus zum proximalen Ende hin, was insbesondere mit der zuvor genannten Ausgestaltung von Vorteil ist, wenn zumindest eine Reihe der Kontaktfinger des Bildsensors nicht an der Außenseite des dritten Abschnitts, sondern auf der Außenseite des ersten und/oder zweiten Abschnitts mit der Platine kontaktiert ist, da dann die Querschnittsabmessung des Bildaufnehmermoduls trotz Kontaktierung der Kontaktfinger an der Außenseite des ersten bzw. zweiten Abschnitts nicht vergrößert wird.

Hinsichtlich des eingangs genannten Verfahrens zum Zusammenbauen eines Bildaufnehmermoduls wird die der Erfindung zugrunde liegende Aufgabe dadurch gelöst, dass die Platine starr ist und mit einer Länge bereitgestellt wird, die kleiner oder gleich der Länge der Kontaktfinger ist, und dass der Raum der Platine in Form einer Nut oder Einkerbung zwischen dem ersten, zweiten und dritten Abschnitt, die miteinander ungelenkig verbunden sind, ausgebildet ist, in die vor dem Anbringen des Bildsensors zumindest ein elektronisches Bauteil eingefügt und mit der Platine an der Außenseite des dritten Abschnitts kontaktiert wird.

Dabei ist es bevorzugt und im Sinne einer stabilen Ausführung der einstückigen Platine vorteilhaft, wenn die Platine vor ihrer Bereitstellung aus einem quaderförmigen, insbesondere etwa würfelförmigen Vollmaterial gefertigt wird, wobei in das Vollmaterial durch Materialausnehmung eine Nut eingebracht wird, wodurch der erste, zweite und dritte Abschnitt der Platine und der Raum zwischen diesen Abschnitten gebildet werden.

Im Fall, dass die Steuerelektronik für den Bildsensor mehr als ein elektronisches Bauteil umfasst, ist es weiterhin bevorzugt, wenn vor dem Anbringen des Bildsensors in dem Raum zwischen dem ersten, zweiten und dritten Abschnitt diese elektronischen Bauteile eingefügt und mit der Platine kontaktiert werden, wobei die zumindest zwei elektronischen Bauelemente auf dem dritten Abschnitt, d.h. innenseitig, in übereinanderliegender Anordnung angebracht werden.

Dabei kann beispielsweise das erste elektronische Bauteil auf die Innenseite des dritten Abschnitts geklebt werden, und das zweite elektronische Bauteil wird dann auf das erste Bauteil geklebt, usw.

In einer weiteren bevorzugten Ausgestaltung wird das zumindest eine elektronische Bauelement mit zumindest einem Leiterdraht an einer Stelle des Raums zwischen dem ersten, zweiten und dritten Abschnitt kontaktiert, wobei dann der Leiterdraht anschließend mit der Außenseite des dritten Abschnitts kontaktiert wird.

Nach dem Einfügen des zumindest einen elektronischen Bauelements nebst dem zumindest einen Leiterdraht wird der verbleibende freie Raum zwischen dem ersten, zweiten und dritten Abschnitt mit einer aushärtenden Füllmasse ausgefüllt.

Da der freie Raum, der in Form einer Nut oder Auskerbung ausgebildet ist, vorzugsweise an zwei Seiten, die quer zum ersten und zweiten Abschnitt verlaufen, offen ist, werden diese Seiten vor dem Einfüllen der Füllmasse verschalt, beispielsweise mit einer Folie, die sich vorzugsweise nach dem Aushärten der Füllmasse von dieser leicht ablösen lässt.

Dabei ist es weiterhin bevorzugt, wenn die Füllmasse vor dem Anbringen des Bildsensors an der Platine in den Raum zwischen den drei Abschnitten eingefüllt wird.

Hierbei ist von Vorteil, dass die Seite der Platine, an der später der Bildsensor angebracht wird, als Einfüllöffnung zum Einfüllen der Füllmasse genutzt werden kann.

In einer weiteren bevorzugten Ausgestaltung werden zumindest die Kontaktfinger einer Reihe des Bildsensors auf der Außenseite des dritten Abschnitts umgelenkt und auf dieser mit der Platine kontaktiert.

Schließlich wird vorzugsweise ein mehradriges Kabel an der Außenseite des dritten Abschnitts mit der Platine kontaktiert, wobei diese Kontaktierung vorzugsweise lösbar ist, wie bereits oben beschrieben wurde.

Weitere Vorteile und Merkmale ergeben sich aus der nachfolgenden Beschreibung und der beigefügten Zeichnung.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ein Ausführungsbeispiel der Erfindung ist in der Zeichnung dargestellt und wird mit Bezug auf diese hiernach näher beschrieben. Es zeigen:
- Fig. 1: eine stark vergrößerte Darstellung in einer Ansicht von schräg distal eines Bildaufnehmermoduls im fertig zusammengebauten Zustand;
- Fig. 2: eine Platine des Bildaufnehmermoduls in Fig. 1 in Alleinstellung in einer Ansicht von schräg distal;
- Fig. 3: einen elektronischen Bildsensor des Bildaufnehmermoduls in Fig. 1 in Alleinstellung in einer Ansicht von schräg distal ;
- Fig. 4: ein elektronisches Bauelement des Bildaufnehmermoduls in Fig. 1 in Alleinstellung in einer Ansicht von schräg distal;
- Fig. 5: einen schritt des Verfahrens zum Zusammenbauen des Bildaufnehmermoduls in Fig. 1 in einer Ansicht von schräg distal;
- Fig. 6: einen weiteren Schritt des Verfahrens zum Zusammenbauen des Bildaufnehmermoduls in Fig. 1 in einer Ansicht von schräg distal;
- Fig. 7: einen noch weiteren Schritt des Verfahrens zum Zusammenbauen des Bildaufnehmermoduls in Fig. 1 in einer Ansicht von schräg distal;
- Fig. 8: einen noch weiteren Schritt des Verfahrens zum Zusammenbauen des Bildaufnehmermoduls in einer Ansicht von schräg proximal;
- Fig. 9: einen noch weiteren Schritt des Verfahrens zum Zusammenbauen des Bildaufnehmermoduls in Fig. 1 in einer Ansicht von schräg proximal; und
- Fig. 10: das fertig zusammengebaute Bildaufnehmermodul nach einem letzten Schritt des Verfahrens zum Zusammenbauen des Bildaufnehmermoduls in einer Ansicht von schräg proximal.

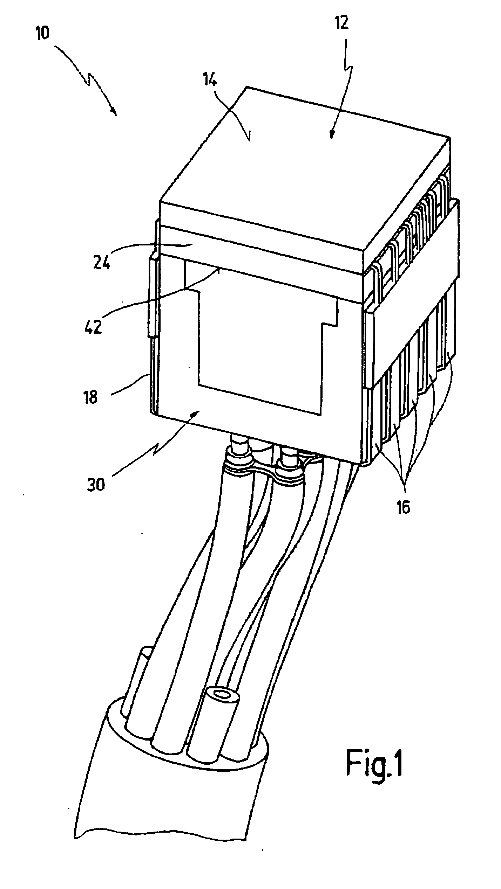

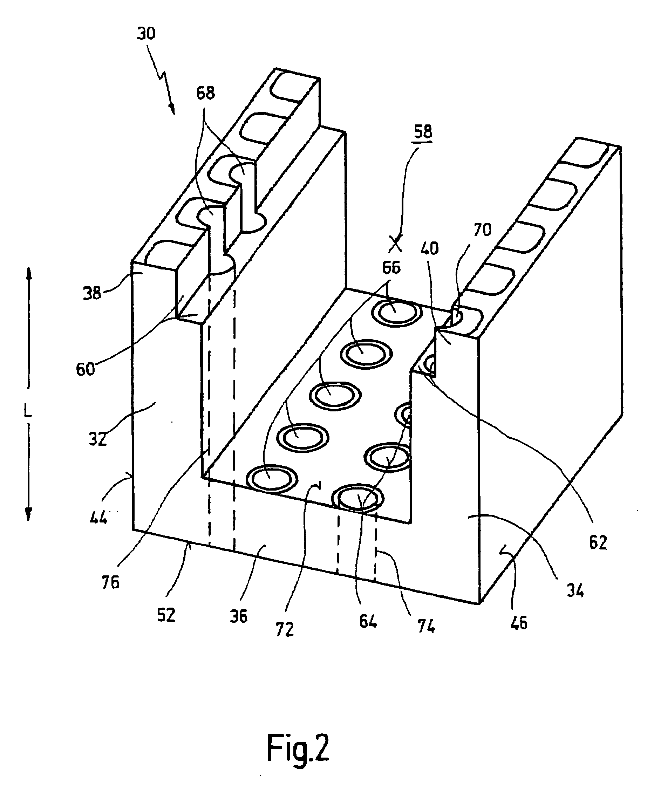

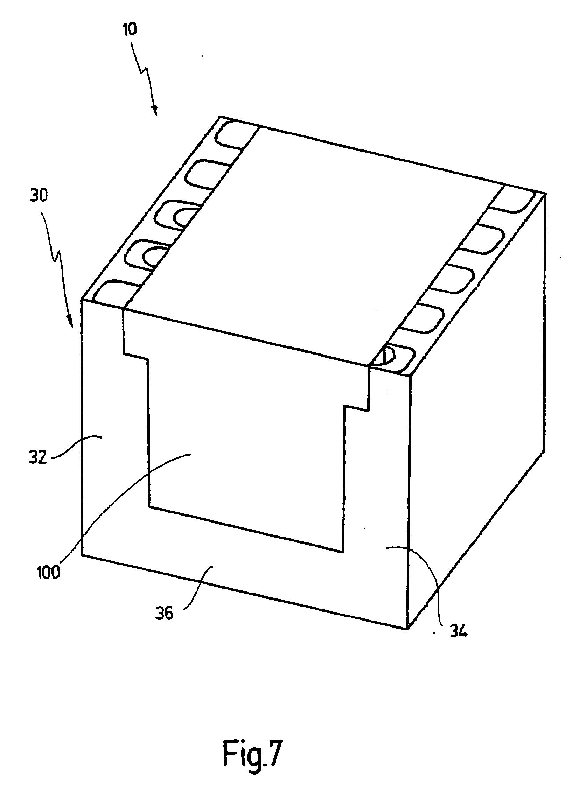

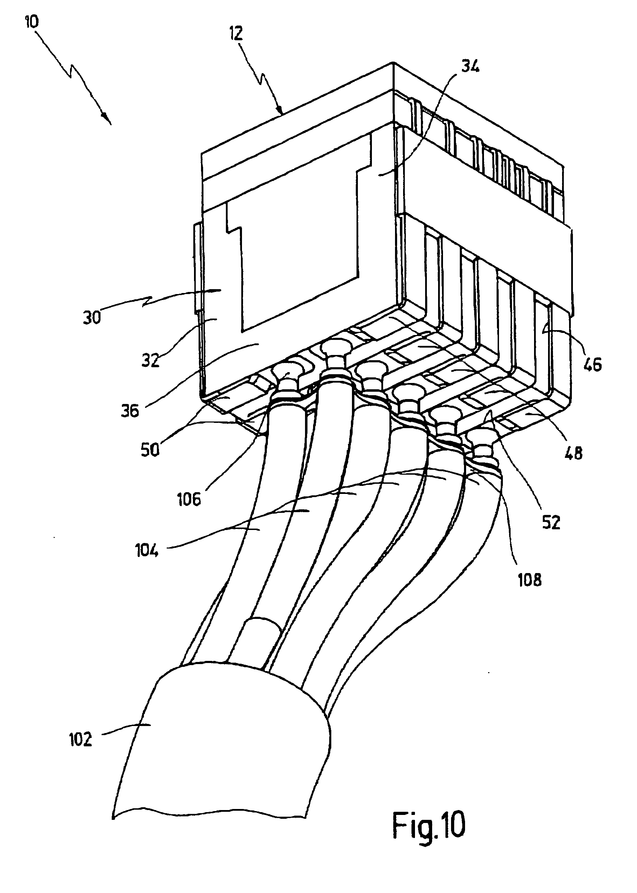

In Fig. 1 und 10 ist ein mit dem allgemeinen Bezugszeichen 10 versehenes Bildaufnehmermodul in seiner Gesamtheit und im fertig zusammengebauten Zustand dargestellt. Einzelheiten des Bildaufnehmermoduls 10 sind in den Fig. 2 bis 9 dargestellt.

Das Bildaufnehmermodul 10 wird vorzugsweise in einem nicht dargestellten Endoskop, insbesondere Videoendoskop, oder in einer miniaturisierten Kamera eingesetzt. Das Bildaufnehmermodul 10 ist ein optoelektronisches Bauteil in miniaturisierter Form. Es versteht sich, dass die Darstellungen in Fig. 1 bis 10 stark vergrößert sind.

Das Bildaufnehmermodul 10 weist als erste Hauptkomponente einen elektronischen Bildsensor 12 auf. Der Bildsensor 12 weist eine lichteintrittsseitige Außenseite 14 auf, durch die Licht in den Bildsensor 12 eintritt. Der lichteintrittsseitigen Außenseite 14 ist im Gebrauch des Bildaufnehmermoduls 10, beispielsweise im in einem Endoskop in dessen distaler Spitze eingebauten Zustand, eine Abbildungsoptik vorgeschaltet, um ein zu beobachtendes Objekt auf dem Bildsensor 12 abzubilden.

Der Bildsensor 12 ist in CCD- oder CMOS-Technologie in TAB-Konfiguration ausgebildet.

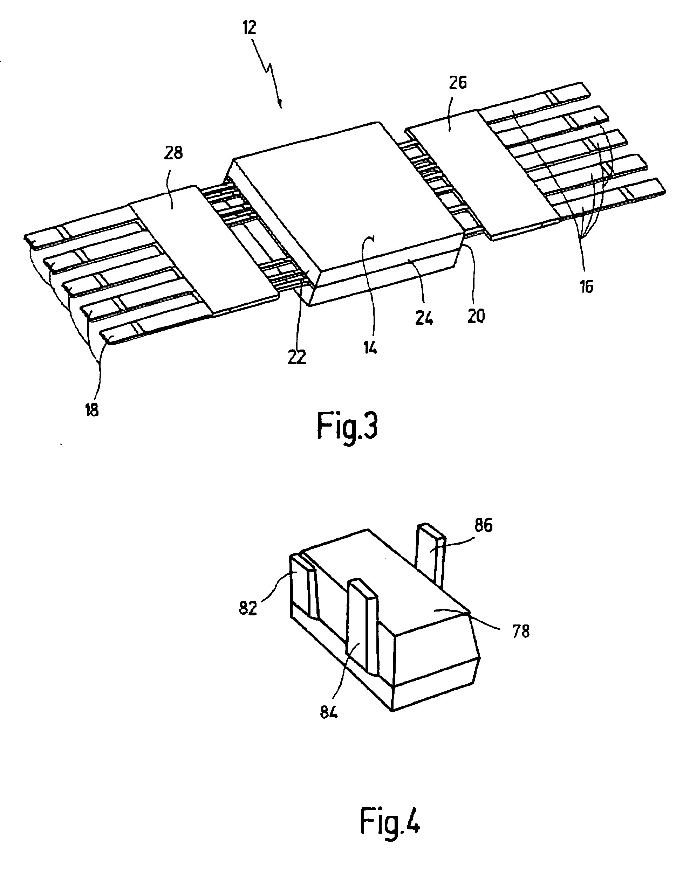

Der Bildsensor 12 weist eine Mehrzahl von Kontaktfingern 16 und 18 auf, im vorliegenden Fall insgesamt zehn solcher Kontaktfinger 16 und 18. Die Kontaktfinger 16 und 18 sind in zwei Reihen an gegenüberliegenden Seiten 20 und 22 eines Basiskörpers 24 des Bildsensors 12 angeordnet. Die Kontaktfinger 16 bilden dabei eine erste Reihe von Kontaktfingern und die Kontaktfinger 18 eine zweite Reihe von Kontaktfingern. In Fig. 3 ist der Bildsensor 12 in Alleinstellung in seinem Ausgangszustand vor Zusammenbau des Bildaufnehmermoduls 10 dargestellt. In diesem Ausgangszustand erstrecken sich die Kontaktfinger 16 und 18 im Wesentlichen parallel zur Ebene des Basisteils 24. Die Kontaktfinger 16 sind über ein nicht leitendes Plättchen 26 miteinander verbunden, und die Kontaktfinger 18 entsprechend über ein nicht leitendes Plättchen 28.

Eine weitere Hauptkomponente des Bildaufnehmermoduls 10 ist eine Platine 30 (Fig. 1), die in Alleinstellung in Fig. 2 dargestellt ist.

Die Platine 30 weist drei ungelenkig und einstückig miteinander verbundene Abschnitte 32, 34 und 36 auf, wobei der erste Abschnitt 32 und der zweite Abschnitt 34 im Wesentlichen parallel verlaufen und voneinander beabstandet sind und sich gemäß Fig. 1 im Wesentlichen quer zum Bildsensor 12 erstrecken, während sich der dritte Abschnitt 36 quer zu dem ersten Abschnitt 32 und dem zweiten Abschnitt 34 und im Wesentlichen parallel zum Bildsensor 12 erstreckt. Gemäß Fig. 1 ist der Bildsensor 12 an Enden 38 und 40 des ersten und zweiten Abschnitts 32 und 34, die dem dritten Abschnitt 36 abgewandt sind, angeordnet und auf diesen ohne Abstand zu den Abschnitten 32 und 34 befestigt, beispielsweise aufgeklebt. Eine der lichteintrittsseitigen Außenseite abgewandte Unter- oder Innenseite 42 ist somit der Platine 30 zugewandt.

Die Kontaktfinger 16 und 18 verlaufen entlang Außenseiten 44 und 46 des ersten Abschnitts 32 bzw. zweiten Abschnitts 34.

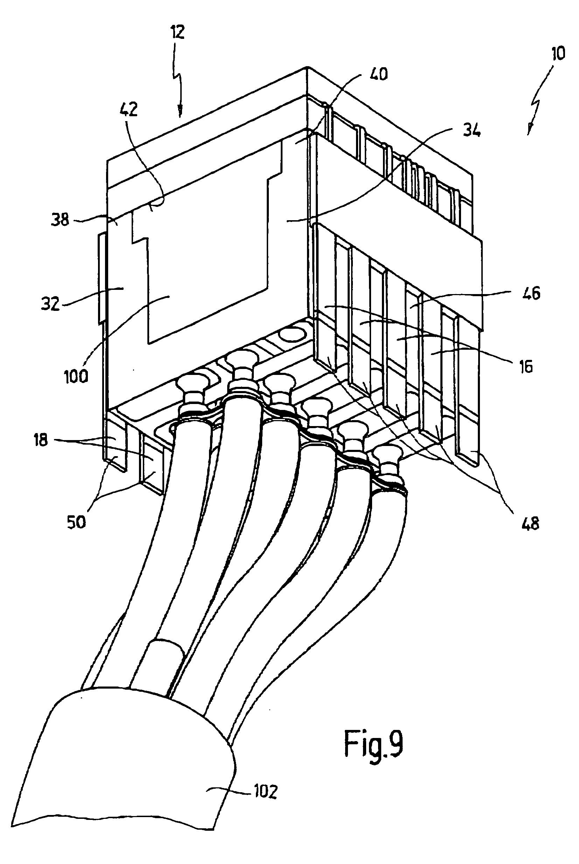

Die Länge L des ersten Abschnitts 32 und des zweiten Abschnitts 34 ist kleiner als die Länge der Kontaktfinger 16 bzw. 18 (vgl. insbesondere Fig. 9), so dass Enden 48 bzw. 50 der Kontaktfinger 16 bzw. 18 die Abschnitte 32 bzw. 34 auf eine Außenseite 52 des dritten Abschnitts 36 greifen, wie in Fig. 10 am besten dargestellt ist.

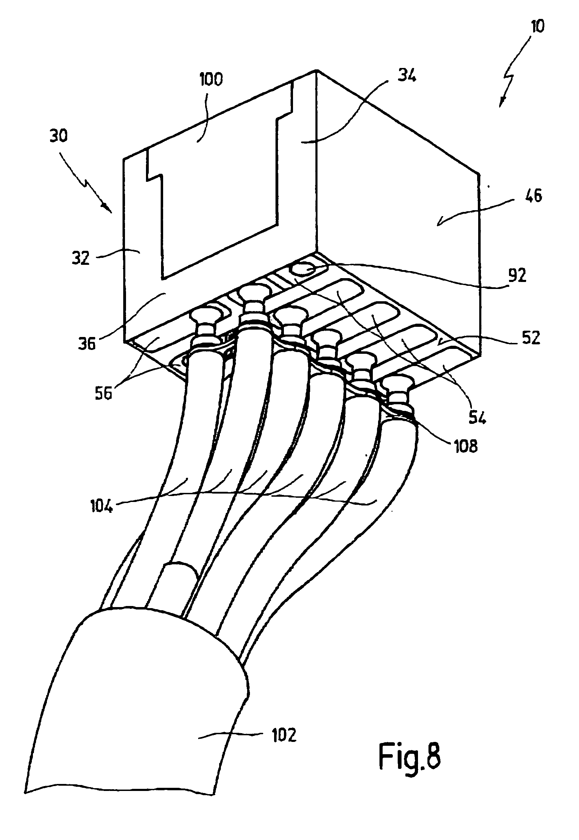

An der Außenseite 52 des dritten Abschnitts 36 sind die Kontaktfinger 16 bzw. 18 elektrisch mit der Platine 30 kontaktiert, die an der Außenseite 52 des dritten Abschnitts entsprechende Kontakte 54 bzw. 56 aufweist (vgl. Fig. 8).

Die Platine 30 ist mit Ausnahme von Kontaktierungsstellen, beispielsweise der Kontakte 54 und 56, insgesamt aus einem nicht leitenden Material, beispielsweise aus Kunststoff, gefertigt.

Zwischen dem ersten Abschnitt 32, dem zweiten Abschnitt 34 und dem dritten Abschnitt 36 weist die Platine 30 einen Raum 58 auf, der zur Aufnahme von elektronischen Bauelementen dient, wie hiernach noch beschrieben wird.

Bei der Fertigung der Platine 30 wird diese ursprünglich aus einem quader- oder würfelförmigen vollmaterial gefertigt, in das eine Nut oder Einkerbung durch Materialausnehmung aus dem Vollmaterial, beispielsweise durch Ausfräsen, eingebracht wird, um den Raum 58 zu schaffen. Die Nut bzw. Einkerbung ist dabei an drei Seiten offen, wie aus Fig. 2 hervorgeht.

Wie aus Fig. 2 hervorgeht, weist der Raum 58 zwischen den Abschnitten 32, 34 und 36 eine T-förmige Struktur auf, wobei sich der breitere Abschnitt in Richtung quer zu den Abschnitten 32 und 34 an deren Enden 38 und 40 befindet. Der Raum 58 weist somit an dem ersten Abschnitt 32 eine Stufe 60 und am zweiten Abschnitt 34 eine Stufe 62 auf.

Die Platine 30 weist ferner einer Mehrzahl von Bohrungen 64, 66, 68 und 70 auf. Die Bohrungen 64 und 66 erstrecken sich durch den dritten Abschnitt 36 von dessen Innenseite 72 bis zu dessen Außenseite 52, wie mit unterbrochenen Linien 74 für eine der Bohrungen 64 angedeutet ist.

Die Bohrungen 68 und 70 erstrecken sich durch den ersten Abschnitt 32 bzw. durch den zweiten Abschnitt 34, und münden im Bereich des Endes 38 bzw. 40 der Abschnitte 32 und 34 an den Stufen 60 bzw. 62, und am gegenüberliegenden Ende ebenfalls auf der Außenseite 52 des dritten Abschnitts 36, wie mit unterbrochenen Linien 76 für eine der Bohrungen 68 in Fig. 2 angedeutet ist. Alle Bohrungen 64 bis 70 sind voneinander durch das Material der Platine 30 isoliert. Innenseitig können die Bohrungen 64 bis 70 elektrisch leitend metallisiert sein, d.h. einen Leiter aufweisen, beispielsweise indem die Wandungen der Bohrungen 64 bis 70 metallisiert sind, oder die Bohrungen 64 bis 70 vollständig mit einem Metall ausgefüllt sind. Auf diese weise stellen die Bohrungen 64 bis 70 Durchkontaktierungen durch den Platinenkörper 30 dar.

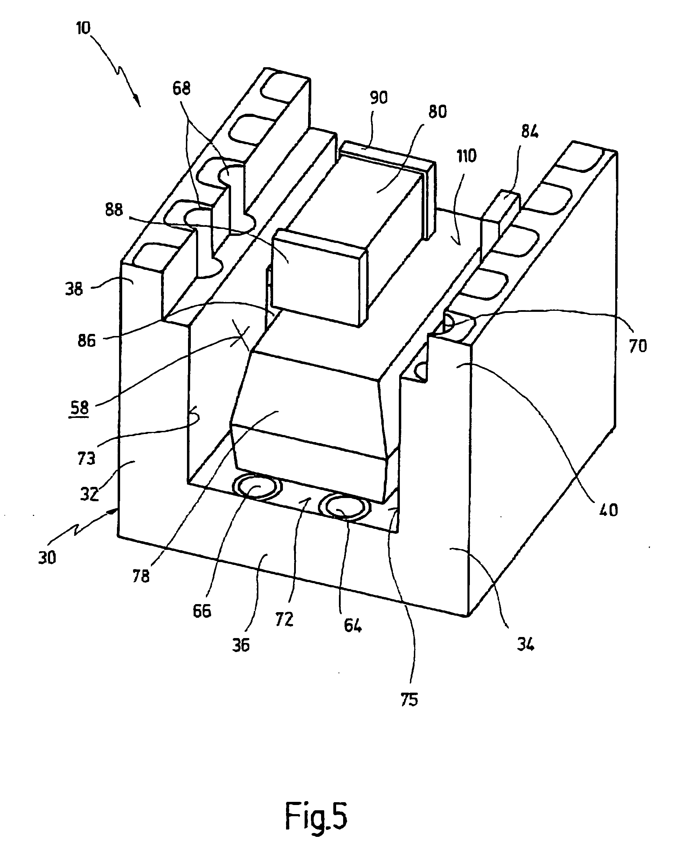

Wie in Fig. 5 dargestellt ist, dient der Raum 58 zwischen dem ersten, zweiten und dritten Abschnitt 32, 34, 36 der Aufnahme von elektronischen Bauelementen für die Steuerelektronik des Bildsensors 12. Im vorliegenden Fall weist das Bildaufnehmermodul 10 ein erstes elektronisches Bauelement 78 und ein zweites elektronisches Bauelement 80 auf. Das erste elektronische Bauelement 78 ist ein Transistor, der in Fig. 4 in Alleinstellung dargestellt ist, und das zweite elektronische Bauelement 80 ist ein Kondensator. Das erste elektronische Bauelement 78 ist auf der Innenseite 72 des dritten Abschnitts 36 zwischen dem ersten und zweiten Abschnitt 32 und 34 angeordnet und auf dieser befestigt, beispielsweise durch Kleben, und das zweite elektronische Bauelement 80 ist auf dem ersten elektronischen Bauelement 78 angeordnet und auf diesem, ebenfalls beispielsweise durch Kleben, befestigt. Somit sind die beiden elektronischen Bauelemente 78 und 80 übereinanderliegend in dem Raum 58 angeordnet, wodurch der Raum 58 optimal in seinen dreidimensionalen Abmessungen genutzt wird.

Das erste elektronische Bauelement 78 weist drei Kontakte 82, 84, 86 auf (vgl. Fig. 4), die von dem elektronischen Bauelement 78 nach oben, d.h. zu den Enden 38 bzw. 40 der Abschnitte 32 und 34 zeigend, abstehen. Der das erste elektronische Bauelement 78 bildende Transistor ist beispielsweise ein handelsüblicher Transistor in miniaturisierter Ausführung, wobei die Kontakte 82, 84, 86 gegebenenfalls vor dem Einbau in die Platine 30 wie in Fig. 4 dargestellt gebogen und teilweise abgelängt werden, um die geeignete Position und Länge für die Kontaktierung des Bauelements 78 zu erreichen.

Das zweite elektronische Bauelement 80 weist zwei Kontakte 88 und 90 auf, die an stirnseiten des Bauelements 80 angeordnet sind.

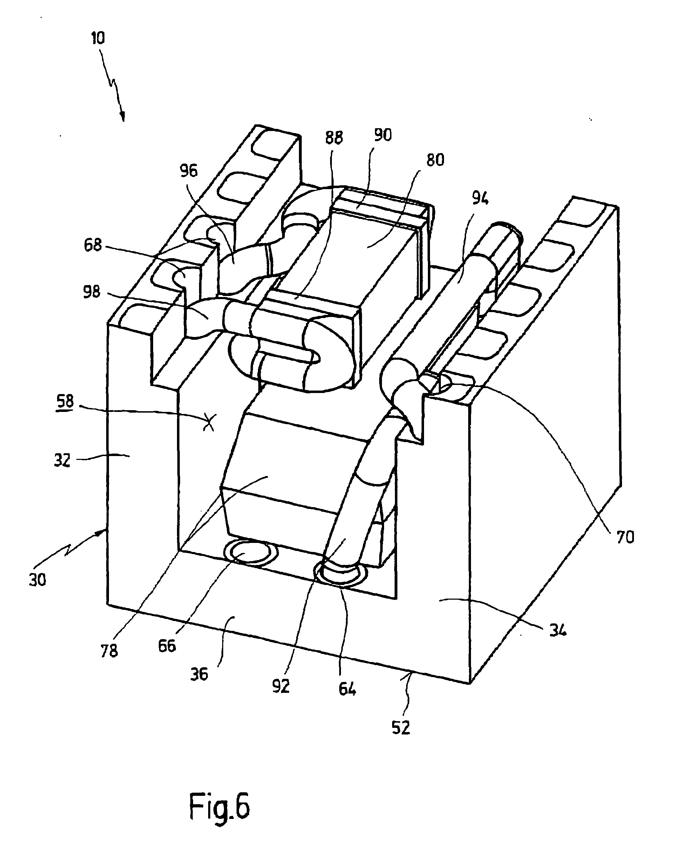

Zur elektrischen Verbindung, d.h. Kontaktierung des ersten und zweiten elektronischen Bauelements 78 und 80 mit der Platine 30, sind gemäß Fig. 6 eine Mehrzahl von Leiterdrähten 92 bis 98 und ein weiterer in der Zeichnung nicht zu sehender Leiterdraht vorgesehen. Entsprechend der Anzahl von insgesamt fünf Kontakten der elektronischen Bauelemente 78 und 80 sind entsprechend fünf derartige Leiterdrähte 92 bis 98 vorgesehen. Die Leiterdrähte 92 bis 98 sind vorzugsweise ohne isolierenden Außenmantel ausgebildet, um Raumbedarf für solche Isolierungen einzusparen. Die Isolierung der Leiterdrähte 92 bis 98 erfolgt auf eine andere, später beschriebene Art und weise.

Die einzelnen Leiterdrähte 92 bis 98 sind an den elektronischen Bauelementen 78 und 80 jeweils an Stellen mit diesen kontaktiert, die von dem ersten, zweiten und dritten Abschnitt 32, 34, 36 beabstandet sind. Auf diese weise erfolgt auch die Kontaktierung der Bauelemente 78 und 80 in optimal platzsparender Weise unter Ausnutzung der Dreidimensionalität des Raums 58 in der Platine 30.

Die Leiterdrähte 92 bis 98 sind durch die Bohrungen 64 bis 70 zur Außenseite 52 des dritten Abschnitts 36 durchgeführt oder zumindest durchkontaktiert. So ist beispielsweise der Leiterdraht 92 durch die Bohrung 64 im dritten Abschnitt 36 bis zur Unterseite 52 des dritten Abschnitts 36 durchgeführt oder durchkontaktiert, wie in Fig. 8 zu sehen ist. Die T-förmige Ausgestaltung des Raums 58 bzw. der Nut 58 hat hier den Vorteil, dass beispielsweise die Leiterdrähte 94, 96 und 98 aus den entsprechenden Bohrungen 68 bzw. 70 austreten können, ohne dass die Gefahr besteht, mit dem darüber befindlichen Bildsensor 12 in Kontakt zu kommen oder das spätere Anbringen des Bildsensors 12 auf den Abschnitten 32 und 34 zu behindern.

Die eigentliche Kontaktierung der Bauelemente 78 und 80 erfolgt aufgrund der Durchführung oder Durchkontaktierung der Leiterdrähte 92 bis 98 auf der Außenseite 52 des dritten Abschnitts 36 der Platine 30, wie bereits zuvor für den Leiterdraht 92 mit Bezug auf Fig. 8 erläutert wurde.

Der verbleibende übrige Bereich des Raums 58 zwischen den Abschnitten 32, 34 und 36 der Platine 30 und den Bauelementen 78 und 80 ist mit einer aushärtenden Füllmasse 100 ausgefüllt, wobei die Füllmasse 100 beispielsweise ein zunächst flüssig vorliegendes Epoxyharz ist, das eine im flüssigen Zustand ausreichende geringe Viskosität aufweist, um in alle verbleibenden freien Zwischenräume und Teilbereiche des Raums 58 zu gelangen. Die Füllmasse 100 bewirkt dabei gleichzeitig eine Isolierung der Leiterdrähte 92 bis 98 und aller elektrisch leitenden Oberflächen, beispielsweise der Kontakte 82 bis 86 des ersten Bauelements 78 und der Kontakte 88 und 90 des zweiten Bauelements 80. In Fig. 1, 7 bis 10 ist die Füllmasse 100 als nicht transparent dargestellt, so dass die sich im Raum 58 befindlichen Bauelemente 78 und 80, die Leiterdrähte 92 bis 98 und die Innenseiten 72, 73 und 75 der Abschnitte 32, 34, 36 in diesen Darstellungen nicht zu sehen sind.

Die Füllmasse 100 kann jedoch ebenso auch transparent ausgebildet sein.

Gemäß Fig. 1, 8, 9 und 10 ist das Bildaufnehmermodul 10 mit einem Kabel 102 verbunden, das eine Mehrzahl von Adern 104 aufweist. Die Adern 104 sind mit der Platine 30 jeweils einzeln kontaktiert, wobei mit 106 eine Kontaktierungsstelle für eine der Adern 104 in Fig. 10 bezeichnet ist. Die Adern 104 sind dabei an der Unterseite 52 des dritten Abschnitts 36 der Platine 30 kontaktiert, ebenso wie die Enden 48 bzw. 50 der Kontaktfinger 16 und 18 des Bildsensors 12.

Ein Abschirmelement 108, das in Form einer Platte ausgebildet ist, ist mit den einzelnen Adern 104 zur elektrischen Abschirmung verbunden.

Das Kabel 102 bzw. die Adern 104 sind vorzugsweise lösbar mit der Platine 30 verbunden. Dies kann in nicht dargestellter Weise dadurch erfolgen, dass eine weitere Platine bereitgestellt wird, die komplementär zur Außenseite 52 des dritten Abschnitts 36 der Platine 30 ausgebildet ist. Die einzelnen Adern werden dann mit dieser zusätzlichen Platine kontaktiert, und die Platine als Ganzes wird mit der Außenseite 52 des dritten Abschnitts 36 der Platine 30 verbunden, beispielsweise durch Löten. Wenn dann das Bildaufnehmermodul 10 von dem Kabel 102 getrennt werden soll, muss nur die Lötstelle oder Klebstelle zwischen der zusätzlichen Platine und der Platine 30 gelöst werden, was einfacher ist, als wenn jede einzelne Ader 104 von der Außenseite 52 des dritten Abschnitts 36 der Platine 30 abgelötet werden muss.

Das Bildaufnehmermodul 10 weist insgesamt eine würfelförmige Struktur auf, wobei die Seitenabmessungen der würfelförmigen Struktur etwa 2 mm x 2 mm oder kleiner sind. Die Außenseiten 44 und 46 des ersten Abschnitts 32 und des zweiten Abschnitts 34 der Platine 30 können sich auch vom Bildsensor 12 aus gesehen nach proximal verjüngen, d.h. zur Mitte des dritten Abschnitts 36 hin geneigt sein. Es können aber auch die Abschnitte 32 und 34 als Ganzes zur Mitte des dritten Abschnitts 36 hin geneigt sein.

Des weiteren können auf den Außenseiten 44 und 46 des ersten Abschnitts 32 und des zweiten Abschnitts 34 der Platine 30 rillenartige Vertiefungen vorgesehen sein, um die Kontaktfinger 16 und 18 darin aufzunehmen, so dass die Kontaktfinger 16 und 18 des Bildsensors 12 auf den Außenseiten 44 und 46 der Abschnitte 32 und 34 der Platine 30 nicht auftragen.

Des Weiteren könnte vorgesehen sein, die Kontaktfinger 16 bzw. 18 des Bildsensors 12 teilweise auch außenseitig an den Abschnitten 32 und 34 mit der Platine 30 zu kontaktieren, so dass auf der Unterseite 52 des dritten Abschnitts 36 der Platine 30 mehr Raum zur Kontaktierung des Kabels 102 bzw. dessen Adern 104 besteht.

Nachfolgend wird noch ein Verfahren zum Zusammenbauen des Bildaufnehmermoduls 10 beschrieben.

Zunächst werden die Platine 30 gemäß Fig. 2 und der Bildsensor 12 gemäß Fig. 3 bereitgestellt.

Die Platine 30 gemäß Fig. 2 kann zuvor aus einem würfelförmigen oder quaderförmigen Vollmaterialstück durch eine Materialausnehmung zur Herstellung des Raums 58 zwischen dem ersten Abschnitt 32, dem zweiten Abschnitt 34 und dem dritten Abschnitt 36 gefertigt werden. Des Weiteren werden die Bohrungen 64, 66, 68, 70 in die Platine 30 eingebracht, gegebenenfalls werden diese Bohrungen mit Metallisierungen wie oben beschrieben versehen.

Beim nächsten Schritt wird zunächst das erste elektronische Bauelement 78 bereitgestellt, wobei gegebenenfalls die Kontakte 82 bis 86 des Bauelements 78 in die richtige Position gemäß Fig. 4 gebogen und gegebenenfalls abgelängt werden.

Anschließend wird gemäß Fig. 5 das erste elektronische Bauelement 78 auf der Innenseite 72 des dritten Abschnitts 36 positioniert und dort befestigt, beispielsweise angeklebt. Das zweite elektronische Bauelement 80 wird auf einer Seite 110, also auf dem elektronischen Bauelement 7B positioniert und an diesem befestigt, beispielsweise durch Kleben.

Im nächsten Schritt werden die Leiterdrähte 92 bis 98, die zuvor in die in Fig. 6 gebrachte Form gebogen werden, mit den Kontakten 82 bis 86 des ersten Bauelements 78 bzw. 88 und 90 des zweiten Bauelements 80 elektrisch leitend verbunden, d.h. kontaktiert (Fig. 6). Im übrigen werden die Leiterdrähte 92 bis 98 durch die Bohrungen 64, 66, 68 und 70 zur Außenseite 52 des dritten Abschnitts 36 durchkontaktiert.

Nachdem die elektronischen Bauelemente 78 und 80 mit der Platine 30 kontaktiert sind, wird der verbleibende Teil des Raums 58 der Platine 30 mit der Füllmasse 100 ausgefüllt, und die Füllmasse 100 wird anschließend ausgehärtet.

Im nächsten Schritt gemäß Fig. 8 wird das Kabel 102 mit den Adern 104 an der Unterseite 52 des dritten Abschnitts 36 kontaktiert. Zuvor wurden die Adern 104 gemäß Fig. 8 freigelegt und mit dem Abschirmelement 108 versehen. Die freien Enden der Adern 104 wurden zuvor ebenfalls entisoliert. Die Kontaktierung der Adern mit der Unterseite 52 des dritten Abschnitts 36 der Platine 30 erfolgt durch eine übliche Kontaktierungstechnik, wie beispielsweise Löten.

Nach der Kontaktierung des Kabels 102 an der Platine 30 wird der Bildsensor 12 mit seiner Unterseite 42 auf die Enden 38 und 40 der Abschnitte 32 und 34 der Platine 30 und die ausgehärtete Füllmasse 100 unmittelbar aufgesetzt, d.h. ohne Abstand zu den Enden 38 und 40. Die Kontaktfinger 16 und 18 des Bildsensors 12 werden etwa rechtwinklig zur Ebene des Bildsensors 12 umgebogen und entlang den Außenseiten 44 und 46 der Abschnitte 32 und 34 angeordnet (Fig. 9).

Schließlich werden im letzten Schritt die Enden 48 und 50 der Kontaktfinger 16 bzw. 18 des Bildsensors 12 auf die Außenseite 52 des dritten Abschnitts 36 der Platine 30 umgebogen, wie in Fig. 10 dargestellt ist, wobei dann die Enden 48 und 50 mit der Platine 30 kontaktiert werden. Nun sind der Bildsensor 12, die elektronischen Bauelemente 78, 80 und das Kabel 102 allesamt auf der Außenseite 52 des dritten Abschnitts 36 der Platine 30 kontaktiert und entsprechend dem vorgegebenen Belegungsplan untereinander elektrisch verbunden.

Die Reihenfolge des Kontaktieren des Kabels 102 und des Anbringen des Bildsensors an der Platine 30 ist vorzugsweise auch in umgekehrter Reihenfolge möglich, d.h. zuerst wird der Bildsensor 12 an der Platine 30 befestigt und die Kontaktfinger 16 und 18 werden mit der Platine kontaktiert, und erst anschließend wird dann das Kabel 102 mit der Platine 30 kontaktiert.

## Patentansprüche

1. Bildaufnehmermodul, insbesondere für ein Endoskop oder eine Miniaturkamera, mit einem elektronischen Bildsensor (12), der eine Mehrzahl von Kontaktfingern (16, 18) aufweist, die in zwei Reihen an gegenüberliegenden Seiten angeordnet sind und jeweils eine Länge aufweisen, und mit einer Platine (30), mit der der die Kontaktfinger (16, 18) elektrisch kontaktiert sind, wobei die Platine (30) zumindest drei einstückig miteinander verbundene Abschnitte (32, 34, 36) aufweist, von denen sich ein erster und ein zweiter Abschnitt (32, 34) voneinander beabstandet im Wesentlichen quer zur Ebene des Bildsensors (12) und ein dritter Abschnitt (36) im Wesentlichen parallel zur Ebene des Bildsensors (12) erstrecken, so dass zwischen dem ersten, zweiten und dritten Abschnitt (32, 34, 36) ein Raum (58) vorhanden ist, wobei der Bildsensor (12) an dem dem dritten Abschnitt (36) abgewandten Ende (38, 40) des ersten und zweiten Abschnitts (32, 34) angeordnet ist, und wobei zumindest eine Reihe der Kontaktfinger (16, 18) des Bildsensors (12) entlang einer Außenseite des ersten oder zweiten Abschnitts (32, 34) verläuft, wobei die Platine (30) starr ist und der erste Abschnitt (32), der zweite Abschnitt (34) und der dritte Abschnitt (36) ungelenkig miteinander verbunden sind, die Länge des ersten Abschnitts (32) und/oder des zweiten Abschnitts (34) kleiner als oder gleich der Länge zumindest eines Teils der Kontaktfinger (16, 18) ist, und der Raum (58) zwischen dem ersten, zweiten und dritten Abschnitt (32, 34, 36) der Platine (30) in Form einer Nut oder Einkerbung ausgebildet ist, in der zumindest ein elektronisches Bauelement (78, 80) für die Steuerelektronik des Bildsensors (12) vorhanden ist.

2. Bildaufnehmermodul nach Anspruch 1, **dadurch gekennzeichnet, dass** die entlang dem ersten oder zweiten Abschnitt (32, 34) verlaufenden Kontaktfinger (16, 18) des Bildsensors (12) zumindest teilweise auf eine Außenseite (52) des dritten Abschnitts (36) der Platine (30) greifen und an dieser mit der Platine (30) kontaktiert sind.

3. Bildaufnehmermodul nach Anspruch 1 oder 2, **dadurch gekennzeichnet, dass** beide Reihen der Kontaktfinger (16, 18) an der Außenseite (44, 46) des ersten Abschnitts (32) und des zweiten Abschnitts (34) vorbeigeführt sind und auf der Außenseite (52) des dritten Abschnitts (36) der Platine (30) mit dieser kontaktiert sind.

4. Bildaufnehmermodul nach einem der Ansprüche 1 bis 3, **dadurch gekennzeichnet, dass** auf der Außenseite (44, 46) des ersten und/oder zweiten Abschnitts (32, 34) Vertiefungen zur Aufnahme der Kontaktfinger (16, 18) ausgebildet sind.

5. Bildaufnehmermodul nach einem der Ansprüche 1 bis 4, **dadurch gekennzeichnet, dass** in dem Raum (58) zumindest zwei elektronische Bauelemente (78, 80) in Richtung von dem dritten Abschnitt (36) zu dem Bildsensor (12) übereinanderliegend angeordnet sind.

6. Bildaufnehmermodul nach einem der Ansprüche 1 bis 5, **dadurch gekennzeichnet, dass** das zumindest eine elektronische Bauelement (78, 80) mit zumindest einem Leiterdraht (92 - 98) an einer Stelle des Raums (58), die von dem ersten, zweiten und dritten Abschnitt (32, 34, 36) beabstandet ist, kontaktiert ist.

7. Bildaufnehmermodul nach Anspruch 6, **dadurch gekennzeichnet, dass** der zumindest eine Leiterdraht (92 - 98) ausgehend von dem zumindest einen elektronischen Bauelement (78, 80) zumindest teilweise durch eine sich durch den ersten, zweiten oder dritten Abschnitt (32, 34, 36) erstreckende Bohrung (64 - 70) geführt ist, die an der Außenseite (52) des dritten Abschnitts (36) mündet.

8. Bildaufnehmermodul nach einem der Ansprüche 1 bis 7, **dadurch gekennzeichnet, dass** der verbleibende freie Teil des Raumes (58) zwischen dem ersten, zweiten und dritten Abschnitt (32, 34, 36) und dem zumindest einen elektronischen Bauelement (78, 80) mit einer isolierenden aushärtbaren Füllmasse (100) ausgefüllt ist.

9. Bildaufnehmermodul nach einem der Ansprüche 1 bis 8, **dadurch gekennzeichnet, dass** die Platine (30) aus einem quaderförmigen, insbesondere etwa würfelförmigen Vollmaterial gefertigt ist, und dass der Raum (58) zwischen dem ersten, zweiten und dritten Abschnitt (32, 34, 36) durch eine Materialausnehmung aus dem Vollmaterial gebildet ist.

10. Bildaufnehmermodul nach einem der Ansprüche 1 bis 9, **dadurch gekennzeichnet, dass** der Raum (58) zwischen dem ersten, zweiten und dritten Abschnitt (32, 34, 36) im Querschnitt etwa die Form eines T aufweist, wobei sich der breitere Abschnitt des Raums (58) an den dem Bildsensor (12) zugewandten Enden (38, 40) des ersten und zweiten Abschnitts (32, 34) befindet.

11. Bildaufnehmermodul nach einem der Ansprüche 1 bis 10, **dadurch gekennzeichnet, dass** der Bildsensor (12) unmittelbar an den Enden (38, 40) des ersten und zweiten Abschnitts (32, 34) der Platine (30) befestigt ist.

12. Bildaufnehmermodul nach einem der Ansprüche 1 bis 11, **dadurch gekennzeichnet, dass** sich durch das Material zumindest eines von dem ersten und dem zweiten Abschnitt (32, 34) der Platine (30) von der Außenseite (52) des dritten Abschnitts (36) ausgehend eine Mehrzahl an Bohrungen, die ggf. als Leiter ausgeführt sind, und/oder Leitern erstrecken, die an einer Innenseite (72) des dritten Abschnitts (36), des ersten und/oder zweiten Abschnitts (32, 34) münden bzw. enden.

13. Bildaufnehmermodul nach einem der Ansprüche 1 bis 12, **dadurch gekennzeichnet, dass** der erste und/oder der zweite Abschnitt (32, 34) zumindest au-ßenseitig zum dritten Abschnitt (36) hin zur Mittelachse des dritten Abschnitts schräg verlaufen.

14. Bildaufnehmermodul nach einem der Ansprüche 1 bis 13, **dadurch gekennzeichnet, dass** es etwa würfelförmig ausgebildet ist und Seitenabmessungen von etwa 2 mm x 2 mm oder kleiner aufweist.

15. Bildaufnehmermodul nach einem der Ansprüche 1 bis 14, **dadurch gekennzeichnet, dass** die Platine (30) mit einem mehradrigen Kabel (102) verbunden ist, und dass das Kabel (102) an der Außenseite (52) des dritten Abschnitts (36) mit der Platine (30) kontaktiert ist.

16. Bildaufnehmermodul nach Anspruch 15, **dadurch gekennzeichnet, dass** das Kabel (102) lösbar mit der Platine (30) verbunden ist.

17. Bildaufnehmermodul nach einem der Ansprüche 1 bis 16, **dadurch gekennzeichnet, dass** zumindest eine Reihe der Kontaktfinger (16, 18) abgelängt und auf einer Außenseite (44, 46) des ersten und/oder zweiten Abschnitts (32, 34) mit der Platine (30) kontaktiert ist.

18. Verfahren zum Zusammenbauen eines Bildaufnehmermoduls (10), wobei ein elektronischer Bildsensor (12) und eine Platine (30) bereitgestellt werden, wobei der Bildsensor (12) eine Mehrzahl von Kontaktfingern (16, 18) aufweist, die in zwei Reihen an gegenüberliegenden Seiten angeordnet sind und eine Länge aufweisen, wobei die Platine (30) zumindest drei Abschnitte (32, 34, 36) aufweist, von denen sich ein erster und ein zweiter Abschnitt (32, 34) voneinander beabstandet im Wesentlichen in einer ersten Richtung und ein dritter Abschnitt (36) in einer zweiten Richtung im Wesentlichen quer zur ersten Richtung erstrecken, so dass zwischen dem ersten, zweiten und dritten Abschnitt (32, 34, 36) ein Raum (58) vorhanden ist, wobei der Bildsensor (12) an dem dem dritten Abschnitt (36) abgewandten Ende (38, 40) des ersten und zweiten Abschnitts (32, 34) angebracht wird, die Kontaktfinger (16, 18) zumindest einer Reihe entlang einer Außenseite (44, 46) des ersten und/oder des zweiten Abschnitts (32, 34) angeordnet und mit der Platine (30) elektrisch kontaktiert werden, wobei die Platine (30) starr ist und mit einer Länge bereitgestellt wird, die kleiner oder gleich der Länge der Kontaktfinger (16, 18) ist, und der Raum (58) der Platine (30) in Form einer Nut oder Einkerbung zwischen dem ersten, zweiten und dritten Abschnitt (32, 34, 36), die miteinander ungelenkig verbunden sind, ausgebildet ist, in die vor dem Anbringen des Bildsensors (12) zumindest ein elektronisches Bauteil (78, 80) eingefügt und mit der Platine (30) kontaktiert wird.

19. Verfahren nach Anspruch 18, **dadurch gekennzeichnet, dass** die Platine (30) vor ihrer Bereitstellung aus einem quaderförmigen, insbesondere etwa würfelförmigen Vollmaterial gefertigt wird, wobei in das Vollmaterial durch Materialausnehmung eine Nut eingebracht wird, wodurch der erste, zweite und dritte Abschnitt (32, 34, 36) der Platine (30) und der Raum (58) zwischen diesen Abschnitten (32, 34, 36) gebildet werden.

20. Verfahren nach Anspruch 18 oder 19, **dadurch gekennzeichnet, dass** vor dem Anbringen des Bildsensors (12) in den Raum (58) zwischen dem ersten, zweiten und dritten Abschnitt (32, 34, 36) zumindest zwei elektronische Bauteile (78, 80) eingefügt und diese mit der Platine (30) kontaktiert werden, wobei die zumindest zwei elektronischen Bauelemente (78, 80) auf dem dritten Abschnitt (36) in übereinanderliegender Anordnung angebracht werden.

21. Verfahren nach einem der Ansprüche 18 bis 20, **dadurch gekennzeichnet, dass** das zumindest eine elektronische Bauelement (78, 80) mit zumindest einem Leiterdraht (92 - 98) an einer Stelle des Raums (58) zwischen dem ersten, zweiten und dritten Abschnitt (32, 34, 36) kontaktiert ist.

22. Verfahren nach einem der Ansprüche 18 bis 21, **dadurch gekennzeichnet, dass** das zumindest eine Bauelement (78, 80) an der Außenseite (52) des dritten Abschnitts (36) kontaktiert wird.

23. Verfahren nach einem der Ansprüche 18 bis 22, **dadurch gekennzeichnet, dass** der nach dem Einfügen des zumindest einen elektronischen Bauelements (78, 80) verbleibende freie Raum (58) zwischen dem ersten, zweiten und dritten Abschnitt (32, 34, 36) mit einer aushärtenden Füllmasse (100) ausgefüllt wird.

24. Verfahren nach Anspruch 23, **dadurch gekennzeichnet, dass** die Füllmasse (100) vor dem Anbringen des Bildsensors (12) an der Platine (30) in den Raum (58) eingefüllt wird.

25. Verfahren nach einem der Ansprüche 18 bis 24, **dadurch gekennzeichnet, dass** zumindest die Kontaktfinger (16, 18) einer Reihe des Bildsensors (12) auf die Außenseite (52) des dritten Abschnitts (36) umgelenkt und auf dieser mit der Platine (30) kontaktiert werden.

26. Verfahren nach einem der Ansprüche 18 bis 25, **dadurch gekennzeichnet, dass** ein mehradriges Kabel. (102) an der Außenseite (52) des dritten Abschnitts (36) mit der Platine (30) kontaktiert wird.

## Claims

1. An image pick-up module, in particular for an endoscope or a miniature camera, comprising an electronic image sensor (12) having a plurality of contact fingers (16, 18) that are arranged in two rows on opposite sides and each have a length, and a circuit board (30), to which the contact fingers (16, 18) are electrically contact-connected, the circuit board (30) having at least three sections (32, 34, 36) which are connected to one another in one piece and of which a first and a second section (32, 34) extend in a manner spaced apart from one another essentially transversely with respect to the plane of the image sensor (12) and a third section (36) extends essentially parallel to the plane of the image sensor (12) so that a space (58) is present between the first, second and third sections (32, 34, 36), the image sensor (12) being arranged at the end (38, 40) of the first and second sections (32, 34) which is remote from the third section (36), and at least one row of the contact fingers (16, 18) of the image sensor (12) running along an outer side of the first or second section (32, 34), wherein the circuit board (30) is rigid and the first section (32), the second section (34) and the third section (36) are connected to one another in an unarticulated manner, the length of the first section (32) and/or of the second section (34) being less than or equal to the length of at least a portion of the contact fingers (16, 18) and the space (58) between the first, second and third sections (32, 34, 36) of the circuit board (30) is configured in the form of a groove or notch, in which at least one electronic component (78, 80) for the control electronics of the image sensor (12) is present.

2. The image pick-up module of Claim 1, **characterized in that** the contact fingers (16, 18) of the image sensor (12) that run along the first or second section (32, 34) at least partly engage on an outer side (52) of the third section (36) of the circuit board (30) and are contact-connected to the circuit board (30) at the said outer side.

3. The image pick-up module of Claim 1 or 2, **characterized in that** both rows of the contact fingers (16, 18) are led past the outer side (44, 46) of the first section (32) and of the second section (34) and are contact-connected to the circuit board (30) on the outer side (52) of the third section (36) of the said circuit board.

4. The image pick-up module of anyone of Claims 1 through 3, **characterized in that** depressions for receiving the contact fingers (16, 18) are formed on the outer side (44, 46) of the first and/or second section (32, 34).

5. The image pick-up module of anyone of Claims 1 through 4, **characterized in that** at least two electronic components (78, 80) are arranged in the space (58) in a manner lying one above the other in the direction from the third section (36) to the image sensor (12).

6. The image pick-up module of anyone of Claims 1 through 5, **characterized in that** the at least one electronic component (78, 80) is contact-connected to at least one conductor wire (92 - 98) at a location of the space (58) which is at a distance from the first, second and third sections (32, 34, 36).

7. The image pick-up module of Claim 6, **characterized in that** the at least one conductor wire (92 - 98), proceeding from the at least one electronic component (78, 80), is at least partly led through a hole (64 - 70) that extends through the first, second or third section (32, 34, 36) and opens at the outer side (52) of the third section (36).

8. The image pick-up module of anyone of Claims 1 through 7, **characterized in that** the remaining free part of the space (58) between the first, second and third section (32, 34, 36) and the at least one electronic component (78, 80) is filled with an insulating curable filling composition (100).

9. The image pick-up module of anyone of Claims 1 through 8, **characterized in that** the circuit board (30) is produced from a parallelepipedal, in particular approximately cuboid, bulk material, and **in that** the space (58) between the first, second and third sections (32, 34, 36) is formed by a material cutout from the bulk material.

10. The image pick-up module of anyone of Claims 1 through 9, **characterized in that** the space (58) between the first, second and third sections (32, 34, 36) has approximately the form of a T in cross section, the wider section of the space (58) being situated at the ends (38, 40) of the first and second sections (32, 34) which face the image sensor (12).

11. The image pick-up module of anyone of Claims 1 through 10, **characterized in that** the image sensor (12) is fixed directly to the ends (38, 40) of the first and second sections (32, 34) of the circuit board (30).

12. The image pick-up module of anyone of Claims 1 through 11, **characterized in that** a plurality of holes, which are embodied as conductors, if appropriate, and/or conductors extend through the material of at least one of the first and second sections (32, 34) of the circuit board (30), proceeding from the outer side (52) of the third section (36), and open or end at an inner side (72) of the third section (36), of the first and/or second section (32, 34).

13. The image pick-up module of anyone of Claims 1 through 12, **characterized in that** the first and/or the second section (32, 34), at least on the outer side, run obliquely with respect to the centre axis of the third section towards the third section (36).

14. The image pick-up module of anyone of Claims 1 through 13, **characterized in that** it is formed in approximately cuboid fashion and has side dimensions of approximately 2 mm x 2 mm or smaller.

15. The image pick-up module of anyone of Claims 1 through 14, **characterized in that** the circuit board (30) is connected to a multi-core cable (102), and **in that** the cable (102) is contact-connected to the circuit board (30) at the outer side (52) of the third section (36).

16. The image pick-up module of Claim 15, **characterized in that** the cable (102) is releasably connected to the circuit board (30).

17. The image pick-up module of anyone of Claims 1 through 16, **characterized in that** at least one row of the contact fingers (16, 18) is cut to length and contact-connected to the circuit board (30) on an outer side (44, 46) of the first and/or second section (32, 34).

18. A method for assembly of an image pick-up module (10), an electronic image sensor (12) and a circuit board (30) being provided, the image sensor (12) having a plurality of contact fingers (16, 18) that are arranged in two rows on opposite sides and have a length, the circuit board (30) having at least three sections (32, 34, 36), of which a first and a second section (32, 34) extend in a manner spaced apart from one another essentially in a first direction and a third section (36) extends in a second direction essentially transversely with respect to the first direction so that a space (58) is present between the first, second and third sections (32, 34, 36), the image sensor (12) being fitted to the end (38, 40) of the first and second sections (32, 34) which is remote from the third section (36), the contact fingers (16, 18) of at least one row being arranged along an outer side (44, 46) of the first and/or of the second section (32, 34) and being electrically contact-connected to the circuit board (30), wherein the circuit board (30) is rigid and provided with a length which is less than or equal to the length of the contact fingers (16, 18), and the space (58) of the circuit board (30) is configured in the form of a groove or notch between the first, second and third sections (32, 34, 36), which are connected to one another in an unarticulated manner, into which groove or notch at least one electronic component (78, 80) is inserted and contact-connected to the circuit board (30) before the image sensor (12) is fitted.

19. The method of Claim 18, **characterized in that** the circuit board (30) prior to its provision, is produced from a parallelepipedal, in particular approximately cuboid, bulk material, a groove being introduced into the bulk material by means of a material cutout, thereby forming the first, second and third sections (32, 34, 36) of the circuit board (30) and the space (58) between these sections (32, 34, 36).

20. The method of Claim 18 or 19, **characterized in that**, before the image sensor (12) is fitted, at least two electronic components (78, 80) are inserted into the space (58) between the first, second and third sections (32, 34, 36) and they are contact-connected to the circuit board (30), the at least two electronic components (78, 80) being fitted on the third section (36) in an arrangement lying one above the other.

21. The method of anyone of Claims 18 through 20, **characterized in that** the at least one electronic component (78, 80) is contact-connected to at least one conductor wire (92 - 98) at a location of the space (58) between the first, second and third sections (32, 34, 36).

22. The method of anyone of Claims 18 through 21, **characterized in that** the at least one component (78, 80) is contact-connected at the outer side (52) of the third section (36).

23. The method of anyone of Claims 18 through 22, **characterized in that** the free space (58) between the first, second and third sections (32, 34, 36) which remains after the insertion of the at least one electronic component (78, 80) is filled with a curing filling composition (100).

24. The method of Claim 23, **characterized in that** the filling composition (100) is filled into the space (58) before the image sensor (12) is fitted to the circuit board (30).

25. The method of anyone of Claims 18 through 24, **characterized in that** at least the contact fingers (16, 18) of one row of the image sensor (12) are diverted onto the outer side (52) of the third section (36) and are contact-connected to the circuit board (30) on the said outer side.

26. The method of anyone of Claims 18 through 25, **characterized in that** a multi-core cable (102) is contact-connected to the circuit board (30) at the outer side (52) of the third section (36).

## Revendications

1. Module de prise de vue, en particulier pour un endoscope ou une caméra miniature, avec un détecteur d'image électronique (12) qui comporte une pluralité de doigts de contact (16, 18) qui sont disposés sur deux rangées sur deux côtés opposés et qui présentent chacun une longueur, et avec une platine (30) avec laquelle les doigts de contact (16, 18) sont en contact électrique, dans lequel la platine (30) comporte au moins trois segments (32, 34, 36) raccordés les uns aux autres d'un seul tenant, dont un premier et un deuxième segment (32, 34) s'étendent à distance l'un de l'autre sensiblement perpendiculairement au plan du détecteur d'image (12) et un troisième segment (36) s'étend sensiblement parallèlement au plan du détecteur d'image (12), de sorte qu'un espace (58) est formé entre le premier, le deuxième et le troisième segment (32, 34, 36), le détecteur d'image (12) étant disposé à l'extrémité (38, 40) du premier et du deuxième segment (32, 34) opposée au troisième segment (36) et au moins une rangée des doigts de contact (16, 18) du détecteur d'image (12) s'étendant le long d'un côté extérieur du premier ou du deuxième segment (32, 34), dans lequel la platine (30) est rigide et le premier segment (32), le deuxième segment (34) et le troisième segment (36) sont raccordés les uns aux autres de manière rigide, la longueur du premier segment (32) et/ou du deuxième segment (34) est égale ou inférieure à la longueur d'au moins une partie des doigts de contact (16, 18) et l'espace (58) entre le premier, le deuxième et le troisième segment (32, 34, 36) de la platine (30) est conçu sous la forme d'une rainure ou d'une encoche dans laquelle est placé au moins un composant électronique (78, 80) pour l'électronique de commande du détecteur d'image (12).

2. Module de prise de vue selon la revendication 1, **caractérisé en ce que** les doigts de contact (16, 18) du détecteur d'image (12) s'étendant le long du premier ou du deuxième segment (32, 34) viennent en prise au moins partiellement sur un côté extérieur (52) du troisième segment (36) de la platine (30) et sont en contact avec la platine (30) sur ce côté.

3. Module de prise de vue selon la revendication 1 ou 2, **caractérisé en ce que** les deux rangées de doigts de contact (16, 18) passent devant le côté extérieur (44, 46) du premier segment (32) et du deuxième segment (34) et, sur le côté extérieur (52) du troisième segment (36) de la platine (30), sont en contact avec celle-ci.

4. Module de prise de vue selon l'une quelconque des revendications 1 à 3, **caractérisé en ce que** des creux sont formés sur le côté extérieur (44, 46) du premier et/ou du deuxième segment (32, 34) pour le positionnement des doigts de contact (16, 18).

5. Module de prise de vue selon l'une quelconque des revendications 1 à 4, **caractérisé en ce que**, dans l'espace (58), au moins deux composants électroniques (78, 80) sont disposés l'un au-dessus de l'autre dans la direction allant du troisième segment (36) vers le détecteur d'image (12).

6. Module de prise de vue selon l'une quelconque des revendications 1 à 5, **caractérisé en ce que** le au moins un composant électronique (78, 80) est en contact avec au moins un fil conducteur (92 - 98) à un emplacement de l'espace (58) qui est éloigné du premier, du deuxième et du troisième segment (32, 34, 36).

7. Module de prise de vue selon la revendication 6, **caractérisé en ce que** le au moins un fil conducteur (92 - 98) partant du au moins un composant électronique (78, 80) passe au moins partiellement à travers un alésage (64 - 70) s'étendant à travers le premier, le deuxième ou le troisième segment (32, 34, 36) qui débouche sur le côté extérieur (52) du troisième segment (36).

8. Module de prise de vue selon l'une quelconque des revendications 1 à 7, **caractérisé en ce que** la partie libre de l'espace (58) restante entre le premier, le deuxième et le troisième segment (32, 34, 36) et le au moins un composant électronique (78, 80) est remplie avec une masse de remplissage isolante durcissable (100).

9. Module de prise de vue selon l'une quelconque des revendications 1 à 8, **caractérisé en ce que** la platine (30) est fabriquée à partir d'une matière pleine parallélépipédique, en particulier à peu près cubique, et **en ce que** l'espace (58) entre le premier, le deuxième et le troisième segment (32, 34, 36) est formé par un enlèvement de la matière pleine.

10. Module de prise de vue selon l'une quelconque des revendications 1 à 9, **caractérisé en ce que** l'espace (58) entre le premier, le deuxième et le troisième segment (32, 34, 36) a, en section transversale, à peu près la forme d'un T, le segment plus large de l'espace (58) se trouvant aux extrémités (38, 40) du premier et du deuxième segment (32, 34) tournées vers le détecteur d'image (12).

11. Module de prise de vue selon l'une quelconque des revendications 1 à 10, **caractérisé en ce que** le détecteur d'image (12) est fixé directement aux extrémités (38, 40) du premier et du deuxième segment (32, 34) de la platine (30).

12. Module de prise de vue selon l'une quelconque des revendications 1 à 11, **caractérisé en ce qu'**une pluralité d'alésages, qui sont éventuellement réalisés sous forme de conducteurs, et/ou de conducteurs qui s'étendent à travers la matière d'au moins l'un des premier et deuxième segments (32, 34) de la platine (30) en partant du côté extérieur (52) du troisième segment (36), débouchent ou se terminent sur un côté intérieur (72) du troisième segment (36), du premier et/ou du deuxième segment (32, 34).

13. Module de prise de vue selon l'une quelconque des revendications 1 à 12, **caractérisé en ce que** le premier et/ou le deuxième segment (32, 34) s'étendent en biais par rapport à l'axe médian du troisième segment au moins sur le côté extérieur par rapport au troisième segment (36).

14. Module de prise de vue selon l'une quelconque des revendications 1 à 13, **caractérisé en ce qu'**il a à peu près une forme cubique et **en ce qu'**il présente des dimensions latérales d'environ 2 mm x 2 mm ou moins.

15. Module de prise de vue selon l'une quelconque des revendications 1 à 14, **caractérisé en ce que** la platine (30) est reliée à un câble multiconducteur (102) et **en ce que** le câble (102) est en contact avec la platine (30) sur le côté extérieur (52) du troisième segment (36).

16. Module de prise de vue selon la revendication 15, **caractérisé en ce que** le câble (102) est relié à la platine (30) de manière amovible.

17. Module de prise de vue selon l'une quelconque des revendications 1 à 16, **caractérisé en ce qu'**au moins une rangée des doigts de contact (16, 18) est coupée à longueur et est en contact avec la platine (30) sur un côté extérieur (44, 46) du premier et/ou du deuxième segment (32, 34).

18. Procédé pour l'assemblage d'un module de prise de vue (10), dans lequel sont proposés un détecteur d'image électronique (12) et une platine (30), dans lequel le détecteur d'image (12) comporte une pluralité de doigts de contact (16, 18) qui sont disposés sur deux rangées sur deux côtés opposés et qui présentent une longueur, dans lequel la platine (30) comporte au moins trois segments (32, 34, 36), dont un premier et un deuxième segment (32, 34) s'étendent à distance l'un de l'autre sensiblement dans une première direction et un troisième segment (36) s'étend dans une seconde direction sensiblement perpendiculaire à la première direction, de sorte qu'un espace (58) est formé entre le premier, le deuxième et le troisième segment (32, 34, 36), le détecteur d'image (12) étant monté à l'extrémité (38, 40) du premier et du deuxième segment (32, 34) opposée au troisième segment (36), les doigts de contact (16, 18) d'au moins une rangée étant disposés le long d'un côté extérieur (44, 46) du premier et/ou du deuxième segment (32, 34) et étant en contact électrique avec la platine (30), dans lequel la platine (30) est rigide et est prévue avec une longueur qui est égale ou inférieure à la longueur des doigts de contact (16, 18), et l'espace (58) de la platine (30) est réalisé sous la forme d'une rainure ou d'une encoche entre le premier, le deuxième et le troisième segment (32, 34, 36) qui sont raccordés les uns aux autres de manière rigide, dans laquelle au moins un composant électronique (78, 80) est inséré et mis en contact avec la platine (30) avant le montage du détecteur d'image (12).

19. Procédé selon la revendication 18, **caractérisé en ce que** la platine (30) est fabriquée à partir d'une matière pleine parallélépipédique, en particulier à peu près cubique, avant sa mise à disposition, une rainure étant formée dans la matière pleine par enlèvement de matière, ce qui permet de former le premier, le deuxième et le troisième segment (32, 34, 36) de la platine (30) et l'espace (58) entre ces segments (32, 34, 36).

20. Procédé selon la revendication 18 ou 19, **caractérisé en ce que**, avant le montage du détecteur d'image (12), au moins deux composants électroniques (78, 80) sont insérés dans l'espace (58) entre le premier, le deuxième et le troisième segment (32, 34, 36) et sont mis en contact avec la platine (30), les au moins deux composants électroniques (78, 80) étant montés l'un au-dessus de l'autre sur le troisième segment (36).

21. Procédé selon l'une quelconque des revendications 18 à 20, **caractérisé en ce que** le au moins un composant électronique (78, 80) est en contact avec au moins un fil conducteur (92-98) à un emplacement de l'espace (58) entre le premier, le deuxième et le troisième segment (32, 34, 36).

22. Procédé selon l'une quelconque des revendications 18 à 21, **caractérisé en ce que** le au moins un composant électronique (78, 80) est en contact sur le côté extérieur (52) du troisième segment (36).

23. Procédé selon l'une quelconque des revendications 18 à 22, **caractérisé en ce que**, après l'insertion du au moins un composant électronique (78, 80), l'espace (58) libre restant entre le premier, le deuxième et le troisième segment (32, 34, 36) est rempli avec une masse de remplissage durcissable (100).

24. Procédé selon la revendication 23, **caractérisé en ce que** la masse de remplissage (100) est versée dans l'espace (58) avant le montage du détecteur d'image (12) sur la platine (30).

25. Procédé selon l'une quelconque des revendications 18 à 24, **caractérisé en ce qu'**au moins les doigts de contact (16, 18) d'une rangée du détecteur d'image (12) sont retournés sur le côté extérieur (52) du troisième segment (36) et sont mis en contact avec la platine (30) sur celui-ci.

26. Procédé selon l'une quelconque des revendications 18 à 25, **caractérisé en ce qu'**un câble multiconducteur (102) est en contact avec la platine (30) sur le côté extérieur (52) du troisième segment (36).
